(19) Europäisches Patentamt
European Patent Office
Office européen des brevets

(11) Publication number: **0 321 100 A2**

(12) **EUROPEAN PATENT APPLICATION**

(21) Application number: **88310976.1**

(51) Int. Cl.⁴: **C07D 337/14 , A61K 31/38**

(22) Date of filing: **21.11.88**

(30) Priority: **10.12.87 CS 9045/87**

(43) Date of publication of application: **21.06.89 Bulletin 89/25**

(84) Designated Contracting States: **AT BE CH DE FR GB IT LI LU NL SE**

(71) Applicant: **SPOFA spojené podniky pro zdravotnickou vyrobu**
**No 11 Husinecká**
**Praha 3(CS)**

(72) Inventor: **Protiva, Miroslav**
**No 7 Bratri Capku**
**Praha 10(CS)**
Inventor: **Jilek, Jiri**
**No 9 Malostranské námesti**
**Praha 1(CS)**
Inventor: **Valchár, Martin**
**no 272 Libcická**
**Praha 8(CS)**
Inventor: **Pomykácek, Josef**
**No 19 Gorazdova**
**Praha 2(CS)**
Inventor: **Dlohozkova, Natasa**
**no 29/537 Kodanská**
**Praha 10(CS)**

(74) Representative: **Fitzpatrick, Alan James et al**
**Fitzpatricks 4 West Regent Street**
**Glasgow G2 1RS Scotland(GB)**

(54) **Piperazine derivatives, their acid addition salts and a process for the preparation of same.**

(57) Pharmaceutically active piperazine derivatives, particularly piperazines substituted in position 1 with a 2-(2-chloro-10,11-dihydrodibenzo(b,f)thiepin-10-yloxy)ethyl residue and in position 4 a hydrogen atom or a methyl, 2-hydroxyethyl, 3-hydroxypropyl, 2-(aminocarbonyl)ethyl, 2-(acetoxy)ethyl or 2 (decanoyloxy)ethyl group, their acid addition salts and processes for the preparation thereof. The compounds are noncataleptic neuroleptics with high affinity to dopamine D-2 receptors and with in vivo antidopaminergic activity but only slight sedative, adrenolytic and anticholinergic properties; but use as antipsychotic drugs free of undesirable side effects. Said compounds are available by several routes, such as (a) reaction of 10-(2-bromo-ethoxy)-2-chloro-10,11-dihydrodibenzo(b,f)-thiepin with piperazine, 1-methylpiperazine, 2-(1-piperazinyl)ethanol, or 3-(1 piperazinyl)propanol and (b) acylation of aminoalcohols obtained by the preceding method with chlorides of the corresponding aliphatic carboxylic acids. Preferred addition salts are succinates, maleates, hydrochlorides and methanesulphonates prepared by the respective neutralization reactions.

EP 0 321 100 A2

# PIPERAZINE DERIVATIVES, THEIR ACID ADDITION SALTS AND A PROCESS FOR THE PREPARATION OF SAME

This invention relates to piperazine derivatives of the general formula I

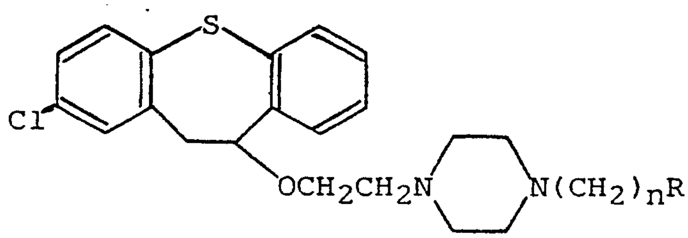

(I)

in which n stands for an integer of 0, 1, 2 or 3 and R represents a hydrogen atom, a hydroxyl or an acyloxy group $O\overset{-}{C}OR^1$ in which $R^1$ is a $C_1$ to $C_9$ alkyl, and their acid addition salts with pharmaceutically acceptable organic or inorganic acids, as well as to a process for their preparation.

The compounds of formula I and their acid addition salts are noncataleptic neuroleptics possessing high affinity to dopamine receptors in brain and markedly pronounced in vivo antidopaminergic activity in respect of their effect on dopamine turnover and metabolism in rat brain striatum. They are of low toxicity, devoid of cataleptic and anti-apomorphine properties (effect on apomorphine stereotypies in rats) and elicit only slight central depressant, incoordinative and adrenolytic action. On the basis of pharmacological assay data the substances are expected to find use in therapy of schizophrenic psychoses; they are substantially free of undesired extrapyramidal, sedative and hypotensive effects.

The following preferred compounds of formula I proved to have favourable pharmacodynamic properties (listed by compound name and dosage form used for pharmacological evaluation respectively).

2-{4-[2-(2-Chloro-10,11-dihydrodibenzo(b,f)thiepin-10-yloxy)ethyl]piperazin 1-yl} ethanol (compound I-A); succinate

3-{4-[2-(2-Chloro-10,11-dihydrodibenzo(b,f)thiepin-10-yloxy)ethyl]piperazin-1-yl} propanol (I-B); bis-(hydrogensuccinate)

1-[2-(2-Chloro-10,11-dihydrodibenzo(b,f)thiepin-10-yloxy)ethyl]-4-methylpiperazine (I-C); bis-(hydrogensuccinate)

1-[2-(2-Chloro-10,11-dihydrodibenzo(b,f)thiepin-10-yloxy)ethyl]piperazine (I-D); succinate

2-{4-[2-(2-Chloro-10,11-dihydrodibenzo(b,f)thiepin-10-yloxy)ethyl]piperazin-1-yl} ethyl acetate (I-E); bis-(hydrogensuccinate)

2-{4-[2-(2-Chloro 10,11-dihydrodibenzo(b,f)thiepin-10-yloxy)ethyl]piperazin-1-yl} ethyl decanoate (I-F); tested as a solution of the base (375 mg/ml) in Miglyol 812 (Trade Mark).

All compounds were evaluated in animals (doses were administered orally) or in vitro, in the aforementioned dosage form; the subsequent dosage data are calculated as the corresponding amounts of the bases.

Acute toxicity in mice, $LD_{50}$ in mg/kg: I-A 346, I-B 618, I-C 401, I-D 509, I-E 691. For comparison, acute toxicity of clozapine (Schmutz J. and Eichenberger E. in Chronicles of Drug Discovery, Vol. 1, by J.S. Bindra and D. Lednicer, editors, published by Wiley, New York 1982, page 39), $LD_{50}$ 199 mg/kg. The inventive compounds thus proved to be substantially less toxic than clozapine, the reference compound.

In the test of inhibition of spontaneous locomotor activity in mice using the photocell method after Dews the medium inhibitory dose $D_{50}$ of compound I-A amounts to 15.7 mg/kg. For other above listed compounds I-B to I-E these $D_{50}$ doses are higher than 10 mg/kg (the given dose elicits a mild but significant inhibition). For clozapine, $D_{50}$ is 1.05 mg/kg. The subject compounds under test are thus at least 10 times less potent central depressants as compared with clozapine; this is a substantial advantage over the reference compound since central depressant properties are undesired.

Compounds I-A to I-E at an oral dose level of 50 mg/kg are completely ineffective in the test for catalepsy in rats and in the test of inhibition of apomorphine stereotypies in the same animals. This is known also to apply for clozapine.

The affinity of the inventive compounds to dopamine D-2 receptors in the rat brain striatum was evaluated on the basis of the inhibition of binding of 0.5 nM $^3$H-spiperone and is expressed as the medium

inhibitory concentration $IC_{50}$ (nM). The respective values are: I-A 212.3, I-B 349.8, I-C 263.7, I-D 1000, I-E 219.5; clozapine 288.5. Compounds I-A, I C and I-E thus possess higher affinity to said dopamine receptors than clozapine.

Antidopaminergic activity of the compounds of this invention was evaluated on the basis of their effect on dopamine turnover and metabolism in rat brain striatum and expressed by increase in the concentration of homovanillic acid, the principal metabolite of dopamine, on oral administration of an 80 mg/kg dose (% concentration of the metabolite in comparison with the control taken for 100 % is given): I-A 504, I-E 244; clozapine 349. Compound I-A is thus more potent antidopaminergic agent than clozapine.

The most potent compound of the invention, compound I-A, assayed following additional biological data. Discoordinating effect (rota-rod test in mice), medium effective dose $ED_{50}$ 44.0 mg/kg, is still lower than for thioridazine (Taeschler M., Cerletti A., Schweiz.Med. Wochschr. 88, 1216, 1958); this is advantageous. In the test of inhibition of apomorphine-induced emesis in dogs the compound had an inhibitory effect at a dosage level of 10 mg/kg and an interval of 4 hours after administration. In the test for antidopaminergic activity threshold doses of I-A which significantly increased homovanillic acid level of tuberculum olfactorium and corpus striatum were respectively 10 and 20 mg/kg. Adrenolytic activity in mice is very low: an oral dose as high as 100 mg/kg prevents adrenaline-induced lethality in 20 % of the animals. In Maccacus rhesus monkeys oral doses of 10 mg/kg do not affect blood pressure, heart rhythm (pulse rate) or general behaviour of the animals. Compound I-A has slight affinity to dopamine D-1 receptors in rat striatum, very low anticholinergic activity both in vitro and in vivo and is only weak inhibitor of re-uptake of serotonin and noradrenaline in rat brain.

Compound I-F has properties of a depot noncataleptic neuroleptic of substantially prolonged action.

From among structures related chemically to compounds of the present invention literature cites their positional isomers of general formula II

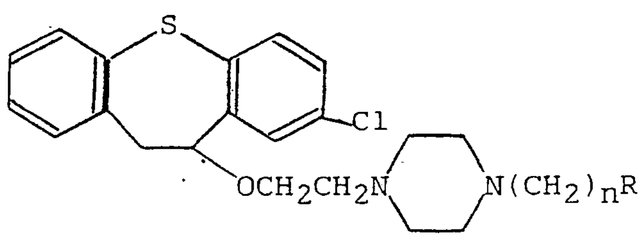

(II)

in which n and R have the same meanings as in formula I except for R being an acyloxy group (Bartl V. et al., Collect.Czech.Chem.Commun. 49, 1810, 1984). These compounds represent neuroleptics of the common profile of activity: they elicit a cataleptic effect in rats ($ED_{50}$ 10 to 15 mg/kg), suppress apomorphine-induced stereotypies in the same animals ($D_{50}$ 2 to 4 mg/kg) and show considerable central depressant activity in the test after Dews ($D_{50}$ approx. 2 mg/kg). These isomeric compounds are thus devoid of all advantages of the subject inventive agents; mere positional shift of the chloro substituent from position 8 to position 2 of the skeleton resulted in this surprising inversion of the neuroleptic activity profile.

The compounds of formula I are available by different preparative routes, more or less general and advantageous. A universal preparative method comprises combining a reactive ester of 2-(2-chloro-10,11-dihydrodibenzo(b,f)-thiepin-10-yloxy)ethanol of general formula III

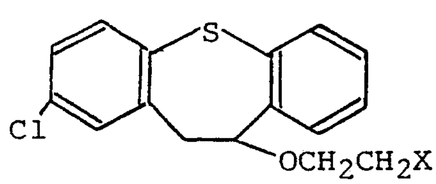

(III)

in which X stands for any halogen atom (except fluorine) or an alkanesulphonyloxy or arenesulphonyloxy group, with a piperazine derivative of general formula IV

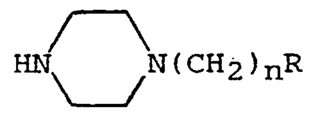

(IV)

in which n and R have the same meanings as in formula I. These reactions can be carried out in various inert solvents in the presence of agents capable of binding the formed hydrogen halide or the respective alkanesulphonic or arenesulphonic acid. The most suitable compound of formula III is 10-(2-bromoethoxy)-2-chloro-10,11-dihydrodibenzo(b,f)thiepin (III, X = Br); convenient alkaline condensing agents are alkali metal carbonates, preferably potassium carbonate, and dimethylformamide proved to be an advantageous reaction medium. The condensations are effected at elevated temperature, preferably within the range of from 90 to 100 °C. Said 10-(2-bromoethoxy) 2-chloro-10,11-dihydrodibenzo(b,f)thiepin, the starting material of choice, is a new compound; its preparation by two alternative procedures is described in Example 1.

Another method of preparing the compounds of formula I comprises the reactions of known 2,10-dichloro-10,11-dihydrodibenzo(b,f)thiepin (Pelz K. et al, Collect.Czech.Chem.Commun. 33, 1852, 1968) with piperazinoethanols of the general formula V

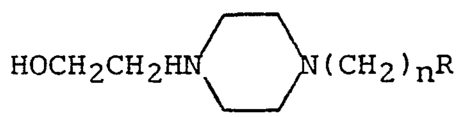

(V)

in which n and R have the same meaning as in formula I with the exception that R does not equal OH and that there is no combination of n = 0 and R = H. This reaction proceeds even at room temperature and is carried out conveniently at the reflux temperature of solvent used, e.g. chloroform. If the piperazine component of formula V is employed in excess, presence of alkaline condensing agents is not necessary; the latter, however, facilitate the reaction course and economize the piperazine component.

Still another method for preparing compounds of formula resides in alkylation of the compound I-D

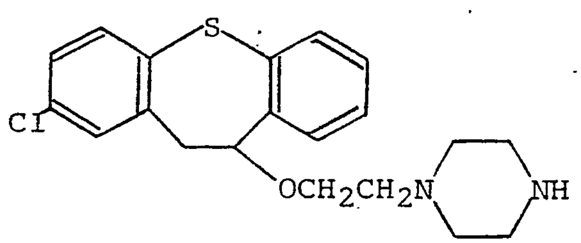

(I-D)

with the respective compounds of formula VI

$X(CH_2)_nR$ (VI)

in which R has the same meaning as in formula I, n is an integer of 1, 2 or 3 and X has the same meaning as in formula III. These alkylations proceed under similar conditions as the principal, first method, conveniently e.g. in dimethylformamide in the presence of potassium carbonate at approx. 100 °C.

Esters of formula I (R = acyloxy group) are advantageously prepared by the acylation of aminoalcohols of formulae I-A or I-B

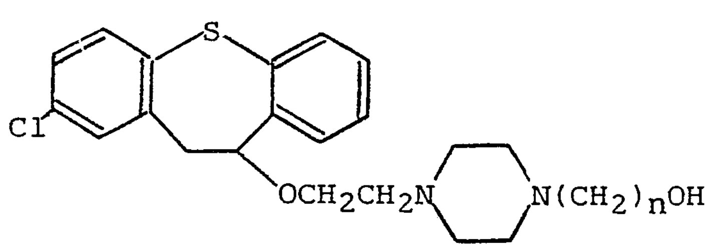

I-A, n = 2
I-B, n = 3

with aliphatic acids or their reactive derivatives of formula VII

$Y\text{-}CO\ R^1$ (VII)

in which $R^1$ has the same meaning as in formula I and Y stands for a hydroxyl, an atom of halogen, preferably chlorine, or an alkoxy group, preferably a methoxyl or an ethoxyl. Besides free carboxylic acids, their chlorides and reactive esters, also acid anhydrides or mixed anhydrides, e.g. mixed anhydrides with monoethyl carbonate, are suitable acylating agents. When acid chlorides are employed, the acylation can conveniently be effected in boiling benzene or chloroform.

The subject compounds are of a basic nature and, by neutralization with pharmaceutically acceptable organic or inorganic acids, yield crystalline salts which are included in the scope of the invention. These salts are more suitable than the corresponding oily bases for the preparation of medicinal dispensing forms and for conducting biological assays. The obtained compounds are racemates, i.e. their structure comprises one centre of chirality. All compounds included herein are new; their identity was verified by analyses and appropriate spectra.

Further particulars of procedures for the preparation of individual compounds of the invention are given in the subsequent examples which illustrate the processes and products of the invention, but are not intended to serve for their exhaustive description.

## Example 1

2-{4-[2-(2-Chloro-10,11-dihydrodibenzo(b,f)thiepin-10-yloxy)ethyl]piperazin-1-yl} ethanol (I-A)

To a stirred solution of 7.5 g of 10 (2-bromoethoxy)-2-chloro-10,11-dihydrodibenzo(b,f)thiepin in 30 ml of dimethylformamide are added 3.5 g of 2-(1-piperazinyl)ethanol and 3.75 g of anhydrous potassium carbonate. The mixture is stirred for 1 hour at room temperature and thereafter for 6 hours at 90 to 92 °C (bath temperature 95 to 100 °C). On standing overnight the mixture is diluted with 65 ml of water and extracted with 100 ml of toluene. The extract is washed with water, filtered with active carbon and the base is extracted from the filtrate into aqueous phase by shaking with a solution of 5 ml of methanesulphonic acid in 75 ml of water. Aqueous solution of the methanesulphonate is filtered with active carbon, the filtrate is alkalized with 12 ml of aqueous ammonia and the liberated base is isolated by extraction with toluene. Processing of the extract by usual procedure yields 7.4 g of the title compound I-A (purity 97 %, yield 85, %).

Neutralization of the obtained base by the respective acids in acetone, aqueous ethanol or an ethanol-ether mixture affords the corresponding addition salts (melting points and solvents for crystallization are given): succinate, 103 to 105 °C (acetone), bis(hydrogen maleate), 165 to 166 °C (aqueous ethanol), dimethanesulphonate, 160 to 162 °C (ethanol-ether), dihydrochloride, 175 to 177 °C (aqueous ethanol).

The starting 10-(2-bromoethoxy)-2-chloro-10,11-dihydrodibenzo(b,f)thiepin used is a new compound, not yet described in literature. This material is available from known compounds by two methods, as illustrated by following procedures A and B.

(A) The solution of 6.0 g of 2-chloro-10,11-dihydrodibenzo(b,f)thiepin-10-ol (Pelz K. et al., l.c.) in 60 ml of benzene is cooled to 12 to 14 °C, under stirring 4.5 g of 2-bromoethanol is added thereto and thereafter within 1.5 hours 3.4 g of boron trifluoride etherate is added dropwise. Stirring at 12 to 14 °C is continued for another hour and the mixture is decomposed by slowly adding 30 ml of water. After 15 minutes of stirring the benzenic layer is separated, washed with water, dried over potassium carbonate and evaporated under reduced pressure. The residue (8.2 g) is crude oily 10-(2-bromoethoxy)-2 chloro10,11-dihydrodibenzo(b,f)thiepin substance containing 95 % of the required compound which can be used in the subsequent procedure; yield of the pure material is 7.8 g (92 %). For accurate characterization a sample is purified by chromatography on silica gel. Elution with petroleum ether containing 10 % of benzene removes less polar components and subsequent elution with a mixture of 70 % of petroleum ether and 30 % of benzene affords the pure compound; its identity is verified by elemental analysis and $^1$H NMR spectrum.

(B) A mixture of 22.0 g of 2-bromoethanol, 5.0 g of potassium carbonate and 5.0 g of 2,10-dichloro-10,11-di hydrodibenzo(b,f)thiepin (Pelz K. et al., l.c.) is stirred for 16 hours at 20 to 22 °C. The mixture is diluted with 10 ml of dichloromethane and refluxed under stirring for 3 hours. On cooling a further 20 ml of dichloromethane is added, inorganic salts are filtered off and washed with the same solvent, volatile components of the filtrate are evaporated under reduced pressure to give an oily residue, crude 10-(2-bromoethoxy)-2 chloro-10,11-dihydrodibenzo(b,f)thiepin of approx. 95 % purity; the yield, based on the pure compound content, is 6.2 g (95 % of theory). The obtained product is identical with the material resulting from method (A); it has density $d_4^{20}$ 1.40 and refractive index $n_D^{20}$ 1.625.

Example 2

3-{4-[2-(2-Chloro-10,11-dihydrodibenzo(b,f)thiepin-10-yloxy)ethyl]piperazin-1-yl} propanol (I-B)

A similar reaction of 15.0 g of 10-(2-bromoethoxy)-2-chloro-10,11-dihydrodibenzo(b,f)thiepin, 7.2 g 3-(1-piperazinyl)propanol (Zawisza T. et al., Acta Pol.Pharm. 22, 477, 1965) and 5.0 g of potassium carbonate in 60 ml of dimethylformamide under conditions substantially of Example 1 yields 15.7 g (86 %) of oily base I-B. Its neutralization with appropriate amounts of succinic acid in acetone affords respectively succinate monohydrate, m.p. 76 to 77 °C (acetone), and bis(hydrogensuccinate), m.p. 71 to 72 °C (acetone).

Example 3

1-[2-(2-Chloro-10,11-dihydrodibenzo(b,f)thiepin-10-yloxy)ethyl]-4-methylpiperazine (I-C)

A similar reaction of 15.0 g of 10-(2-bromoethoxy)-2-chloro-10,11-dihydrodibenzo(b,f)thiepin, 5.0 g of 1-methylpiperazine and 5.0 g of potassium carbonate in 60 ml of dimethylformamide as in Example 1 gives 14.2 g (90 %) of oily base I-C. Its neutralization with succinic acid in aqueous acetone results in crystalline bis(hydrogensuccinate), m.p. 116 to 117 °C (aqueous acetone).

Example 4

1-[2-(2-Chloro-10,11-dihydrodibenzo(b,f)thiepin-10-yloxy)ethyl]piperazine (I-D)

To a solution of 13.7 g of anhydrous piperazine in 45 ml of dimethylformamide is added 5.5 g of potassium carbonate and within 30 minutes a solution of 15.0 g of 10-(2-bromoethoxy)-2-chloro-10,11-dihydrodibenzo(b,f)thiepin in 15 ml of the same solvent is introduced dropwise under stirring. The mixture is warmed for 6 hours at 100 °C, on cooling it is distributed between 150 ml of water and 150 ml of benzene. The benzenic layer is washed with water and basic products are transferred into the aqueous phase by shaking with 150 ml of 10 % aqueous solution of methane-sulphonic acid. The obtained solution of methanesulphonates is alkalized with 30 ml of aqueous ammonia, the liberated bases are isolated by extraction with benzene, the extract is evaporated and the nonhomogeneous residue (13.7 g) is chromatographed on a column with 300 g of silica gel (Kieselgel 40). Elution with chloroform alone and chloroform with 10 % of ethanol removes less polar components (8.0 g of stereoisomeric dialkylated piperazines). Oily base I-D (5.7 g, 38 %) is obtained by subsequent elution with chloroform saturated with aqueous ammonia. Neutralization reactions yield, e.g., dihydrochloride monohydrate, m.p. 152 to 154 °C (ethanol-ether), and succinate, m.p. 149 to 150 °C (EtOH).

Example 5

2-{4 [2-(2 Chloro-10,11-dihydrodibenzo(b,f)thiepin-10-yloxy)-ethyl]piperazin-1-yl} ethyl acetate (I-E)

To a stirred solution of 10.5 g of compound I-A (cf. Example 1) in 50 ml of benzene there is dropped within 20 minutes 11.0 g of acetyl chloride. The mixture is stirred for 6 hours at 60 °C, on cooling the formed thick suspension is distributed between 100 ml of water and 100 ml of benzene, the whole is shaken with 30 ml of aqueous ammonia, the benzenic layer is washed with water, dried and evaporated to yield 10.6 g (91 %) of homogeneous ester I-F in the form of a glassy solid. Its neutralization affords crystalline bis(hydrogenmaleate), m.p. 171 to 172 °C (aqueous acetone) and bis(hydrogen-succinate), m.p. 97 to 98

°C (aqueous acetone).

Example 6

2-{4-[2-(2 Chloro-10,11-dihydrodibenzo(b,f)thiepin-10-yloxy)ethyl]piperazin-1-yl} ethyl decanoate (I-F)

(A) To a solution of 10.0 g of compound I-A (cf. example 1) in 40 ml of benzene is dropped under stirring a solution of 6.9 g of decanoylchloride (Fierz-David H.E., Kuster W., Helv.Chim.Acta 22, 82, 1939) in 30 ml of the same solvent and the mixture is refluxed for 8 hours. On cooling it is diluted with 80 ml of benzene, a solution of 10 ml of concentrated aqueous ammonia in 100 ml of water is added and the mixture is shaken. The formed emulsion is centrifuged and the separated benzenic layer is dried and evaporated to give 11.0 g (80 %) of oily base I-F. Neutralization of 2.1 g of this base with 0.90 g of maleic acid in acetone yields 2.75 g of its bis(hydrogenmaleate), m.p. 165 °C (aqueous acetone). Pure base for pharmacological evaluation is obtained by decomposition of the salt with dilute aqueous ammonia, extraction with benzene and processing of the extract by procedures known per se.

(B) To a solution of 14.6 g of compound I-A (cf. Example 1) in 50 ml of chloroform a solution of 10.0 g of decanoylchloride in 30 ml of the same solvent is added under stirring within 20 minutes. The mixture is stirred for 2 hours at room temperature and refluxed for 4 hours. On cooling the clear solution is diluted with 50 ml of chloroform and shaken with a mixture of 15 ml of aqueous ammonia and 100 ml of water; the separation of phases proceeds better than in the preceding case. The chloroform portion is dried and evaporated to give 21.0 g (90 %) of nearly homogeneous base I-F. Neutralization with 8.8 g of maleic acid in 350 ml of acetone yields 23.8 g of the bis(hydrogenmaleate), m.p. 158 to 159 °C. After crystallization from a mixture of 1.5 l. of acetone and 25 ml of water the pure salt (18.0 g) melts at 163 to 165 °C. Decomposition with 30 ml of aqueous ammonia in 150 ml of water and extraction with benzene gives 12.5 g (63 %) of the homogeneous base I-F which is suitable for pharmacological evaluation.

## Claims

1. Piperazine derivatives of the general formula I

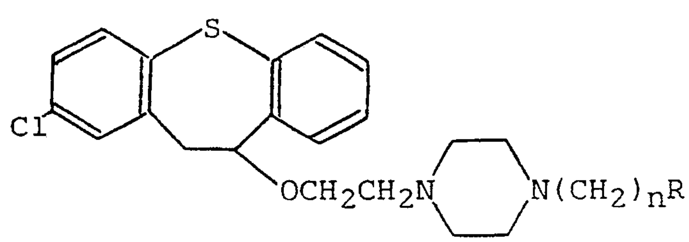

(I)

in which n stands for an integer of 0, 1, 2 or 3 and R represents a hydrogen atom, a hydroxyl or an acyloxy group OCOR[1] with R[1] for a $C_1$ to $C_9$ alkyl, and their acid addition salts with pharmaceutically acceptable organic or inorganic acids.

2. 2- 4 [2-(2-Chloro-10,11-dihydrodibenzo(b,f)thiepin-10-yloxy)ethyl]piperazin-1-yl ethanol and its succinate.

3. 10-(2-bromoethoxy)-2-chloro-10,11-dihydrodibenzo(b,f)thiepin.

4. A process for the preparation of piperazine derivatives of the general formula I

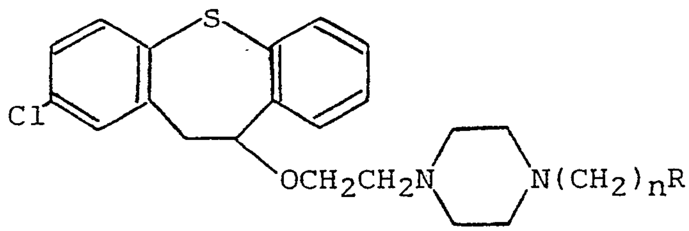

(I)

in which n stands for an integer of 0, 1, 2 or 3 and R represents a hydrogen atom, a hydroxyl or an acyloxy group $O\bar{C}OR^1$ with $R^1$ for a $C_1$ to $C_9$ alkyl, and their acid addition salts comprising reaction of reactive esters of 2-(2-chloro-10,11-dihydrodibenzo(b,f)thiepin-10-yloxy)ethanol of the general formula III

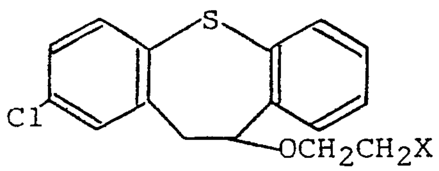

(III)

in which X is any halogen atom except fluorine or an alkaneor arenesulphonyloxy group, with piperazine derivatives of the general formula IV

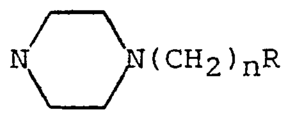

(IV)

in which n and R have the same meanings as in formula I and optionally subjecting the resulting aminoalcohols of formulae I-A or I-B

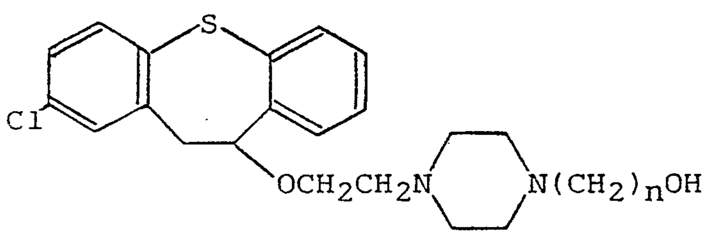

I-A, n = 2
I-B, n = 3

to subsequent acylation with aliphatic carboxylic acids or their reactive derivatives of formula VII
Y-CO-R'. (VII)
in which R' has the same meaning as in formula I and Y stands for a hydroxyl, halogen or alkoxyl, and neutralization of the obtained base with a pharmaceutically acceptable organic or inorganic acid.

5. A process according to claim 4 wherein 10-(2 bromoethoxy)-2-chloro-10,11-dihydrodibenzo(b,f)thiepin is reacted with piperazine, 1-methylpiperazine, 2-(1-piperazinyl)ethanol or 3-(1-piperazinyl)propanol in the presence of potassium carbonate in dimethylformamide at a temperature of from 90 to 100 °C.

6 A process according to claim 4 or claim 5, wherein aminoalcohols of formulae I-A or I-B are acylated with alkanecarbonylchlorides of formula $R^1COCl$, in which $R^1$ has the same meaning as in formula I, in benzene or chloroform.

7. A process according to claim 6 wherein the acylation is carried out at the reflux temperature of the reaction mixture.